# EUROPÄISCHE PATENTSCHRIFT

(11) **EP 0 777 489 B1**
(45) Veröffentlichungstag und Bekanntmachung des Hinweises auf die Patenterteilung: **01.08.2001**
(21) Anmeldenummer: 96919682.3
(22) Anmeldetag: 07.05.1996
(51) Int. Cl.: A61K 35/72, C12P 1/02

(54) **NIEDERMOLEKULARER WIRKSTOFFEXTRAKT AUS HEFEN UND VERFAHREN ZU SEINER HERSTELLUNG**
LOW MOLECULAR YEAST ACTIVE SUBSTANCE EXTRACT AND PROCESS FOR PREPARING THE SAME
EXTRAIT A FAIBLE POIDS MOLECULAIRE DE PRINCIPES ACTIFS DE LEVURES ET SON PROCEDE DE PREPARATION

(30) Priorität: 10.05.1995 DE 19517020
(43) Veröffentlichungstag der Anmeldung: 11.06.1997
(73) Patentinhaber: Thymopharma AG, 6005 Luzern (CH)
(72) Erfinder: JAEGER, Christa, CH-6005 Luzern (CH); JAEGER, Cornelius, CH-6005 Luzern (CH)
(74) Vertreter: Siewers, Gescha, Dr.
(86) Internationale Anmeldenummer: EP9601886
(87) Internationale Veröffentlichungsnummer: WO9635439

(56) Entgegenhaltungen:
- EP-A- 0 065 246
- EP-A- 0 286 033
- GB-A- 2 175 804
- JOURNAL OF BACTERIOLOGY, Bd. 169, Nr. 12, 1987, Seiten 5622-5625, XP000577059 BARNES C.A. ET AL: "Production of heat shock protein is independent of cell cycle blockage in the yeast Saccharomyces cerevisiae"
- CELLULAR AND MOLECULAR BIOLOGY VOL 38(2), 135-143, 1992

## Beschreibung

Die Erfindung betrifft Niedermolekulare, stoffwechselaktivierende Wirkstoffgemische aus Hefepilzen der Ordnung Saccharomycetes, gekennzeichnet durch einen erhöhten Gehalt an Hitzeschockproteinen, erhältlich durch Extraktionsschritte aus Hefen der Ordnung Saccharomycetes, die einige Stunden bei 37 - 38°C kultiviert, anschließend bis auf maximal 45°C erwärmt und dann wieder auf 25°C abgekühlt, zerkleinert, mit einem proteolytischen Enzym aufgeschlossen, zentrifugiert, das Sediment ggf. einmal gewaschen und der Überstand filtriert wird.

Hefeextrakte aus Hefepilzen der Ordnung Saccharomycetes sind ebenso wie ihre anabole und atmungsfördernde sowie allgemein stoffwechselaktivierende Wirkung bekannt. Verfahren zur Gewinnung derartiger Extrakte sind beispielsweise in der EP-B 0065246 beschrieben. Bei diesem Verfahren wird eine Suspension der Hefezellen nach Zusatz eines proteolytischen Enzyms beim Optimum der Gärung einer Ultraschallbehandlung unterzogen, bis die Temperatur der Suspension merklich angestiegen ist. Der Einsatz von Ultraschall zur Dialyse der Hefezellen ist zwar ein wirksames Mittel zur Gewinnung der Extrakte, aber es hat sich herausgestellt, daß die Ultraschallbehandlung durchaus negative Nebenwirkungen zeigt, da praktisch alle Verbindungen in wäßriger Lösung dabei einer mehr oder weniger ausgeprägten Oxidation unterliegen. Bei der Behandlung mit Ultraschall werden also keine genuinen Wirkstoffe aus den Zellen extrahiert, sondern es liegen in der Regel chemisch veränderte Verbindungen vor.

Aus der EP-A-286033 ist ein Präparat aus Saccharomyces bekannt, bei dem eine Hefesuspension mit einem Gehalt an Zuckern bei 15 - 25°C mit einem herkömmlichen CO₂-Laser bestrahlt und danach kurz auf eine Temperatur über 50°C erwärmt wird. Sowohl das Gesamtprodukt als auch Überstand und Rückstand getrennt sind stoffwechselwirksam, aber über die Zusammensetzung des Proteingehaltes ist nichts einzeln bekannt, außer daß sich aufgrund der Beschreibung der Kultivierungsbedingungen mit Sicherheit sagen läßt, daß in einem derartigen Produkt keine Hitzeschockproteine enthalten sein können. Es ist aber aus Cellular and Molecular Biology, Vol. 38 (2) bekannt, daß die Bildung von Hitzeschockproteinen erst mit steigenden Temperaturen zunimmt. Ein bestimmtes Verfahren wird in dieser Vorveröffentlichung nicht beschrieben. Aus Journal of Bacteriology, 169 (12), 1987, 5622-5625 sind radioaktiv markierte Proteine aus Saccharomyces bekannt, die ³² S-Methionin enthalten, aber nach einem anderen Verfahren gewonnen wurden.

Es besteht daher noch ein Bedarf nach Hefeextrakten und Verfahren zu ihrer Herstellung, die zu einem möglichst genuinen Produkt führen und einen möglichst geringen Gehalt an durch das Herstellungsverfahren veränderten Substanzen aufweisen.

Zur Lösung der Aufgabe werden Hefeextrakte nach Anspruch 1 vorgeschlagen sowie Verfahren zu ihrer Herstellung entsprechend Anspruch 2.

Überraschenderweise hat sich gezeigt, daß sich Hefeextrakte mit stoffwechselaktivierender Wirkung in qualitativ und quantitativ verbesserter Form herstellen lassen, wenn die Kulturen in bestimmter Weise einem Temperaturwechsel unterworfen werden. Erfindungsgemäß werden die Hefen, die vor Ansetzen der Kultur bei etwa 15°C gehalten werden, in Wasser suspendiert, wobei das Verhältnis von Hefe : Wasser etwa 1 : 2 beträgt. Der Suspension wird dann ein für die jeweilige Hefe vergärbarer Zucker, insbesondere Saccharose, in Mengen von 5 Gew.-% beigemischt. Die Masse wird dann auf die optimale Kultivierungstemperatur von 37° bis 38°C erwärmt und mindestens 3 Stunden bei dieser Temperatur belassen, wobei in bestimmten Intervallen kurz durchgerührt und belüftet wird. Die Generationszeit der Hefen beträgt etwa 60 Minuten.

Nach 3-stündiger Kultivierung wird die Masse dann relativ schnell bis maximal 45°C erwärmt und 60 Minuten bei dieser Temperatur belassen. Daran anschließend läßt man die Suspension während einer Zeitdauer von 60 Minuten wieder langsam auf 25°C abkühlen und hält sie etwa 60 Minuten bei dieser Temperatur. Die gesamte Zeitdauer des Herstellungsverfahrens beträgt daher etwa 6 Stunden.

Durch die Erhöhung der Kultivierungstemperatur auf maximal 45°C während einer Generationszeit wird die Temperaturresistenz der Hefen verbessert und der Gesamtstoffwechsel deutlich erhöht. Es wird angenommen, daß die erhöhte Ausbeute an niedermolekularen stoffwechselaktiven Wirkstoffen auf die verstärkte Metabolisierung während der erhöhten Wuchstemperatur zurückzuführen ist. Diese Annahme wird dadurch gestützt, daß im Wirkstoffkonzentrat Hitzeschockproteine, nachweisbar sind. Hitzeproteine, abgekürzt hsp sind Proteine, die von lebenden Zellen bei thermischer oder chemischer Belastung in stark erhöhten Raten vorübergehend synthetisiert werden. Ihre genaue Funktion ist noch nicht bekannt, aber sie sind zum Überleben von Streßsituationen essentiell und man vermutet, daß sie die ATP-abhängige Rückfaltung von denaturierten Proteinen initireren bzw. unterstützen. In den Hefehydrolysaten sind Proteine der hsp-Familie 60 und 70 enthalten. Isolierung und Nachweis von hsp sind beispielsweise in Nature (London) 337, 655 bis 659 und 44 bis 47 (1989) beschrieben.

Als Hefen werden bevorzugt Saccharomyces cerevisiae, auch in verschiedenen Reinzuchtformen, oder Saccharomyces uvarum oder Saccharomyces rosei eingesetzt.

Nach der etwa insgesamt 6-stündigen Kulturzeit wird die Masse zerkleinert mit Hilfe geeigneter mechanischer Mühlen wie beispielsweise Kolloidmühlen. Die Zerkleinerung kann ggf. auch im Anschluß an den Enzymzusatz durchgeführt werden. Dann wird die Masse bei etwa 37° bis 38°C mit einem proteolytischen Enzym oder einer Mischung aus solchen Enzymen versetzt, wobei das Verhältnis von Enzym : Biomasse etwa 0,03 : 1 betragen sollte. Die Einwirkungszeit ist von der Art des verwendeten proteolytischen Enzyms abhängig und beträgt in der Regel etwa 180 Minuten. Als proteolytische Enzyme werden vorzugsweise Papain, Ficin oder Bakterien- oder Pilzproteasen eingesetzt.

Nach dem Aufschluß wird die gesamte Suspension innerhalb von 30 Minuten auf 85°C zur Enzyminaktivierung erhitzt und 30 Minuten bei dieser Temperatur belassen. Nach dem Abkühlen wird die Masse zentrifugiert, wobei das Sediment ggf. noch einmal gewaschen werden kann. Der Überstand, der die gesuchten Wirkstoffe enthält, wird filtriert und bei Temperaturen nicht über 40°C im Vakuum eingeengt. Diese Lösung kann anschließend der üblichen und bekannten Sprühtrocknungsgranulierung unterzogen werden.

Die erfindungsgemäßen Wirkstoffgemische weisen gegenüber den bisher bekannten, nach anderen Verfahren hergestellten Wirkstoffgemischen eine deutlich gesteigerte Stoffwechselaktivität auf, die in der Regel im Fibroblastentest deutlich, zum Teil um 50 bis 100% über der bisher bekannter Produkte liegt.

Die Wirkstoffgemische gemäß Erfindung können in der Humanmedizin und in der Vetrinärmedizin überall dort eingesetzt werden, wo eine Stoffwechselaktivierung notwendig ist, beispielsweise zur Förderung der Wundheilung von schlecht heilenden Wunden, zur besseren Nahrungsverwertung, insbesondere auch in der Tier- und Fischzucht. Ein weiteres Einsatzgebiet liegt im Bereich der Stimulation der Aktivität von Mikroorganismen bei enzymatischem Prozessen bei der Bearbeitung von Lebensmitteln.

Im folgenden wird die Erfindung anhand eines Beispieles näher erläutert:

10 kg Saccharomyces cerevisiae der untergärigen Zuchtform werden mit 20 kg gereinigtem und filtriertem Wasser von Trinkwasserqualität in einem Reaktor versetzt. Diese Suspension wird mit 5 Gew.-% Saccharose versehen und unter Rühren gut vermischt. Die Suspension wird dann auf 37° bis 38°C erwärmt und mindestens 3 Stunden bei dieser Temperatur belassen, wobei in etwa halbstündigen Abständen kurz gerührt und belüftet wird. Daran anschließend wird die Masse auf 45°C erwärmt und 60 Minuten bei dieser Temperatur belassen und dann wieder während einer Zeitspanne von 60 Minuten auf 25°C abgekühlt und hierbei 60 Minuten belassen.

Daran anschließend wird die ausgewogene Suspension mit einer entsprechenden Menge Papain versetzt, wobei das Verhältnis von Enzym : Biomasse 0,03 : 1 betragen sollte. Nach Enzymzusatz unter gleichzeitiger Zerkleinerung der Hefen wird die Mischung bei 37° bis 38°C 180 Minuten gerührt. Die Mischung wird dann innerhalb von 30 Minuten auf 85°C erhitzt und 30 Minuten bei dieser Temperatur belassen und dann abgekühlt und zentrifugiert. Das Zentrifugat wird abgezogen und das Sediment noch einmal gewaschen und nochmals zentrifugiert. Die Zentrifugate werden vereint und dann nach dem Filtrieren bei Temperaturen unter 40°C eingeengt, vorzugsweise mit Hilfe eines Vakuumverdampfers. Die eingeengte zellfreie Lösung wird dann in an sich bekannter Weise einer Spühtrocknungsgranulierung unterzogen.

## Patentansprüche

1. Niedermolekulare, stoffwechselaktivierende Wirkstoffgemische aus Hefepilzen der Ordnung Saccharomycetes, gekennzeichnet durch einen erhöhten Gehalt an Hitzeschockproteinen, erhältlich durch Extraktionsschritte aus Hefen der Ordnung Saccharomycetes, die einige Stunden bei 37 - 38°C kultiviert, anschließend bis auf maximal 45°C erwärmt und dann wieder auf 25°C abgekühlt, zerkleinert, mit einem proteolytischen Enzym aufgeschlossen, zentrifugiert, das Sediment ggf. einmal gewaschen und der Überstand filtriert wird.

2. Verfahren zur Herstellung eines niedermolekularen, stoffwechselaktivierenden Wirkstoffgemisches aus Hefen der Gattung Saccharomycetes, bei dem Hefen bei einer Temperatur von 37 - 38°C einige Zeit kultiviert und beim Aufarbeiten zentrifugiert, das Sediment ggf. einmal gewaschen und der Überstand filtriert wird, dadurch gekennzeichnet, daß die Hefen in einem ersten Schritt bei 37 - 38°C kultiviert, dann die Temperatur auf maximal 45°C erhöht und die Kultur einige Zeit hierbei belassen, sodann langsam wieder auf 25°C abgekühlt und bei dieser Temperatur einige Zeit belassen, zerkleinert, mit einem proteolytischen Enzym aufgeschlossen und durch Zentrifugieren, ggf. einmal Waschen und Abfiltrieren des Überstandes aufgearbeitet wird.

3. Verfahren nach Anspruch 2, dadurch gekennzeichnet, daß die Kultur 2 - 6 Stunden bei einer Temperatur von 37 - 38°C belassen, dann auf maximal 45°C erwärmt und etwa 40 - 80 Minuten bei dieser Temperatur belassen, anschließend innerhalb von 40 - 80 Minuten wieder auf 25°C abgekühlt und etwa 40 - 80 Minuten bei dieser Temperatur belassen wird.

4. Verfahren nach Anspruch 2 oder 3, dadurch gekennzeichnet, daß der Suspension während der Kulitivierung ein vergärbarer Zucker in Mengen von 5% beigegeben wird.

5. Verfahren nach Anspruch 2 oder 3, dadurch gekennzeichnet, daß nach dem zweiten Kulturschritt bei 37 - 38°C ein enzymatischer Aufschluß und Zerkleinerung bei dieser Temperatur unter Verwendung von proteolytischen Enzymen erfolgt, daß die Masse dann zur Enzyminaktivierung auf über 80°C erhitzt, dann zentrifugiert und der Überstand in an sich bekannter Weise weiterverarbeitet wird.

6. Verfahren nach Anspruch 2 bis 5, dadurch gekennzeichnet, daß als proteolytisches Enzym Papain verwendet wird.

7. Verfahren nach Anspruch 2 bis 5, dadurch gekennzeichnet, daß das Verhältnis von proteolytischen Enzymen zu Substrat etwa 0,03 : 1, bezogen auf das Gewicht, ist.

8. Verfahren nach Anspruch 2 bis 5, dadurch gekennzeichnet, daß als Hefe Saccharomyces cerevisiae eingesetzt wird.

## Claims

1. Low-molecular, metabolism-activating active-substance mixture of yeast fungi of the order Saccharomycetes, characterised by an increased content of thermal-shock proteins. obtainable from yeasts of the order Saccharomycetes by extraction steps. which is cultured for a few hours at 37-38° C, then heated to a maximum of 45° C and subsequently cooled to 25° C, reduced to small particles, broken down with a proteolytic enzyme, centrifuged, the sediment is washed once if necessary and the supernatant filtered.

2. A method of manufacturing a low-molecular, metabolism-activating active-substance mixture of yeasts of the order Saccharomycetes, in which yeasts are cultured for a time at a temperature of 37-38° C and centrifuged during working up, the sediment is washed once if necessary and the supernatant filtered, characterised in that in a first step the yeasts are cultured at 37-38° C, the temperature is then raised to a maximum of 45° C and the culture kept at this temperature for a time, then slowly cooled again to 25° C and kept at this temperature for a time, reduced to small particles, broken down with a proteolytic enzyme and worked up by centrifuging, washing once if necessary and filtering off the supernatant.

3. A method according to Claim 2, characterised in that the culture is kept at a temperature of 37-38° C for 2-6 hours, then heated to a maximum of 45° C and kept at this temperature for about 40-80 minutes, then cooled again to 25° C within 40-80 minutes and kept at this temperature for about 40-80 minutes.

4. A method according to Claim 2 or 3, characterised in that a fermentable sugar is added to the suspension during culturing in proportions of 5%.

5. A method according to Claim 2 or 3, characterised in that, after the second culturing step at 37-38° C, enzymatic breakdown and particle reduction occurs at this temperature with the use of proteolytic enzymes, in that the mass is then heated to over 80° C to inactivate the enzyme, then centrifuged, and the supernatant is then processed further in a way known per se.

6. A method according to Claim 2 to 5, characterised in that papain is used as the proteolytic enzyme.

7. A method according to Claim 2 to 5, characterised in that the ratio of proteolytic enzymes to substrate is about 0.03 : 1 by weight.

8. A method according to Claim 2 to 5, characterised in that Saccharomyces cerevisiae is used as the yeast.

## Revendications

1. Mélange de principes actifs de faible poids moléculaire ayant une activité métabolique provenant de levures appartenant au genre des Saccharomyces, caractérisé par le fait qu'il a une teneur accrue en protéines de choc thermique, que l'on peut obtenir par des étapes d'extraction de levures appartenant au genre des Saccharomyces, impliquant la culture des levures pendant quelques heures à une température de 37 - 38 °C, le chauffage à une température au plus égale à 45 °C, le refroidissement à 25 °C, le broyage, la digestion par des enzymes protéolytiques, la centrifugation, éventuellement le lavage du culot, et la filtration du surnageant.

2. Procédé de préparation d'un mélange de principes actifs de faible poids moléculaire ayant une activité métabolique provenant de levures appartenant au genre des Saccharomyces, dans lequel on cultive des levures pendant un certain temps à une température de 37 - 38 °C, on les sépare par centrifugation, on lave éventuellement une fois le culot et on filtre le surnageant, caractérisé par le fait que l'on cultive les levures dans une première étape à une température de 37 - 38 °C, que l'on augmente la température à au plus 45 °C et que l'on maintient la culture pendant un certain temps à cette température, que l'on laisse ensuite refroidir lentement jusqu'à 25 °C et que l'on maintient la culture pendant un certain temps à cette température, que l'on broie, que l'on expose les levures à une digestion par des enzymes protéolytiques, et que l'on purifie ensuite par centrifugation, suivie éventuellement d'un lavage, et filtration du surnageant.

3. Procédé selon la revendication 2, caractérisé par le fait que l'on laisse 1a culture pendant 2 à 6 heures à une température de 37 - 38 °C, que l'on chauffe ensuite à au plus 45 °C et que l'on maintient la culture pendant environ 40 - 80 minutes à cette température, que l'on refroidit ensuite sur une période de 40 à 80 minutes jusqu'à 25 °C, et que l'on maintient la culture à cette température pendant environ 40 - 80 °C.

4. Procédé selon la revendication 2 ou 3, caractérisée par le fait que l'on ajoute à la suspension pendant l'incubation 5 % d'un sucre fermentescible.

5. Procédé selon la revendication 2 ou 3, caractérisé par le fait que la deuxième étape d'incubation à 37 - 38 °C est suivie d'une digestion enzymatique et d'un broyage à cette même température en présence d'enzymes protéolytiques, que l'on chauffe ensuite la masse à une température supérieure à 80 °C pour activer les enzymes, que l'on centrifuge et que l'on purifie le surnageant d'une manière connue en soi.

6. Procédé selon l'une des revendications 2 à 5, caractérisé par le fait que l'on utilise la papaïne comme enzyme protéolytique.

7. Procédé selon l'une des revendications 2 à 5, caractérisé par le fait que le rapport pondéral enzyme protéolytique/substrat est d'environ 0,03:1.

8. Procédé selon l'une des revendications 2 à 5, caractérisé par le fait que l'on utilise comme levures des *Saccharomyces cerevisiae.*
